# EUROPEAN PATENT SPECIFICATION

(11) **EP 0 788 807 B1**
(45) Date of publication and mention of the grant of the patent: **04.06.2003**
(21) Application number: 97200306.5
(22) Date of filing: 04.02.1997
(51) Int. Cl.: A61M 25/01

(54) **Controlled flexible catheter**
Steuerbarer flexibler Katheter
Cathéter flexible dirigeable

(30) Priority: 06.02.1996 NL 1002254
(43) Date of publication of application: 13.08.1997
(73) Proprietor: Cordis Corporation, Miami Lakes Florida 33014 (US)
(72) Inventor: Korteling, Bart-Jan, 9301 VR Roden (NL)
(74) Representative: 't Jong, Bastiaan Jacob

(56) References cited:
- EP-A- 0 301 288
- EP-A- 0 415 553
- EP-A- 0 630 657

## Description

The invention relates to a catheter as defined in the introductory part of claim 1.

Such a catheter is known from EP-A-0 630 657. By applying a tensile force to the pull element the flexible end-section will assume a required curved shape.

In the manufacturing of a catheter of the known kind, the connection of the sleeve to the basic body is complicated. Therefor the invention has for its object to provide a catheter of the kind said forth above in which the sleeve is connected to the basic body in an efficient manner.

According to the invention this object is achieved with the features of the characterising portion of claim 1. In the manufacture of the catheter of the invention the tube like basic body and the catheter end section can be simply pushed on the push-on sections of the plugs.

Further advantageous embodiments are provided by the features as defined in the sub claims.

The invention will be explained in greater detail in the following description with reference to the figures.
- Figure 1: shows partly schematically a catheter according to the invention.
- Figure 2: shows a longitudinal section of a catheter according to an embodiment of the invention in a straight shape.
- Figure 3: shows an end section of a catheter according to a further embodiment in a curved shape.

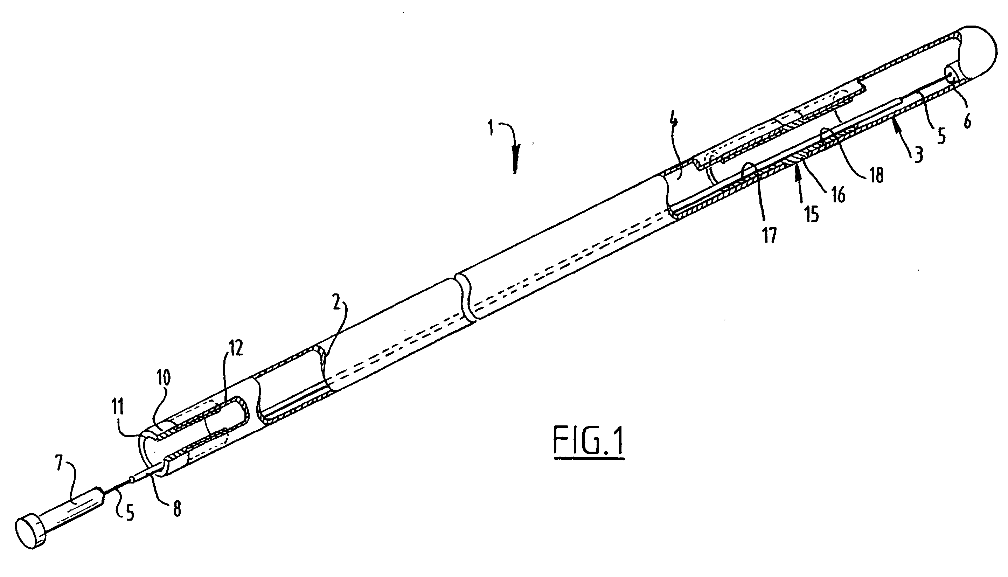

The catheter 1 illustrated in figure 1 comprises a tube-like basic body 2 with a distal end illustrated on the right-hand side and a proximal end illustrated on the left-hand side of the figure.

The catheter 1 comprises in this embodiment one lumen 4. The basic body has an end-section 3 which is relatively flexible in order to be able to bend it in a certain required shape.

This bending is controlled from the proximal end by means of a pull element 5 which extends through the lumen and is fixed at the distal end to the basic body by means of an end-connection 6.

The intended curved shape can for instance be "preprogrammed" by a suitable distribution of bending-stiff and less bending-stiff material in a longitudinal and circumferential direction in the end-section 3. A relatively simple curve can already be achieved in the manner illustrated in the figure by having the pull element 5 engage eccentrically.

According to the invention the pull element 5 is surrounded by a sleeve 8 along part of its length. In the embodiment shown this sleeve fits closely around the pull element 5. The sleeve is connected with the basic body at least close to the proximal end of the basic body 2 and the relatively proximal end of the end-section 3. The reactive compressive force due to the force applied to the pull element 5 is taken up for at least a significant degree by this sleeve, so that the section of the basic body 2 positioned in between the connecting points of the sleeve is subjected to little or no compressive force and can consequently not be deformed by such a compressive force.

With the embodiment shown the sleeve 8 is connected to the basic body by means of plugs 10, 15. At the proximal end the plug 10 comprises a central section 11 which has substantially the same diameter as the basic body 2 and, extending from that, a push-on section 12 which has a somewhat smaller diameter. The outside diameter of the push-on section 12 is substantially the same as the inside diameter of the basic body, which may have been adapted for that purpose.

At the relatively proximal end of the flexible end-section 3 a similar construction has been employed. The plug 15 arranged here also comprises a central section 16 which has substantially the same diameter as the basic body 2 but carries on either side a push-on section 17, 18 respectively. On the push-on section 18 the flexible end-section 3 has been arranged.

When manufacturing the catheter 1, the sleeve 8 can be connected each time in a reliable and properly reproducible manner with the plugs 10 and 15 respectively, before the plugs 10, 15 are connected to the basic body or the end-section 3 thereof respectively. The section of the basic body 2 extending in between the plugs 10 and 15 may be shorter than the corresponding section of the sleeve 8. Consequently the sleeve 8 will lie in between these connecting points with some slack, as a result of which a possible decrease in length due to the reactive compressive force does not lead to the introduction of a compressive force in the corresponding section of the basic body.

The basic body may be connected in the usual manner with the push-on sections of the plugs 10, 15, for instance by glueing or ultrasonic welding when the plugs 10, 15 have been made of a plastic material. When these plugs have been made of metal, suitable joining techniques for that particular combination of materials such as glueing, pressing and shrinking may be used.

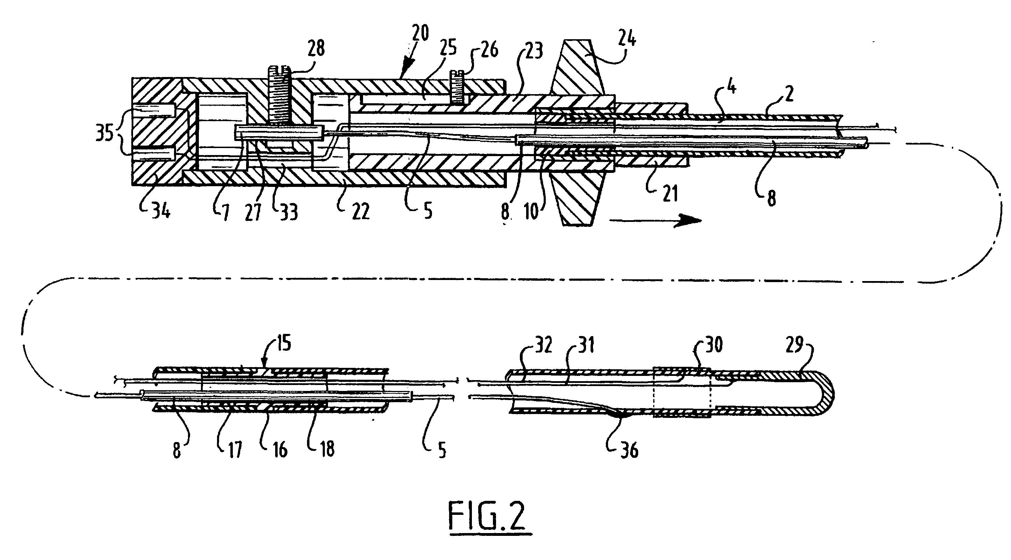

In Figure 2 a catheter according to the invention is shown in operational state, connected with a handle 20 at the proximal end. With this handle 20 the tensile force applied to the pull element 5 can be set.

In Figure 2 parts corresponding to those shown in Figure 1 are indicated with the same reference numerals.

The catheter of Figure 2 is intended for measuring electrical activities specifically in the tissues internally of the heart. For this purpose the catheter body 2 at its distal end is provided with a tip 29 of electrically conductive material, such as metal. A short distance relatively proximal to this end electrode 29 a ring electrode 30 also of electrically conductive material is arranged around the catheter body.

The end electrode 29 and the ring electrode 30 are connected by means of electrical lines 31, 32 with terminals 35 of connector 34 arranged on the handle 20. The lines 31, 32 extend through the lumen 4 of the catheter body 2 and through the handle 20.

For proper measurement of the electrical activities it is necessary that the two electrodes 29, 30 are in proper contact with the tissue of which these electrical activities are to be measured. According to the invention the tip portion of the catheter, carrying the two electrodes 29, 30 can be properly manoeuvred against the tissue to be measured by bending this tip portion in a controlled manner, by exerting a controlled pulling force on the pull element 5.

So as to be able to exert this pulling force in a controlled manner, the proximal end portion of the catheter is mounted in the handle 20 which can be manipulated by the person performing the treatment.

The catheter body 2 at its proximal end is provided with a strain relief 21 which is arranged on the catheter body and the plug 10 by glueing or ultrasonic welding, known as such. This proximal end portion of the catheter is received in a recess arranged in a piston 23 of the handle 20. This piston 23 is slidable in a corresponding bore in the housing 22. The stroke of the piston 23 relative to the housing 22 is limited by means of a stop screw 26 arranged in the housing 22 and extending into a slot 25 arranged in the piston 23.

In the housing 22 a bore 27 is arranged in which an engagement shaft 7 connected to the pull element 5 is received. The engagement shaft 7 is fixed in the bore 27 by means of the set screw 28.

A further bore 33 is arranged in the housing 22 for guiding the signal lines 31, 32 coming from the electrodes 29, 30 to the terminals 35 of the connector 34.

On the relative distal end of the piston 23 an engaging ring is fixed, which can be engaged by the thumb and index finger while the housing 22 is held with the remaining fingers in the palm of the hand.

When holding the handle 20 in this way, a very carefully controlled pulling force can be exerted on the pull element 5 relative to the catheter body 2, by pushing the engaging ring 24 forward.

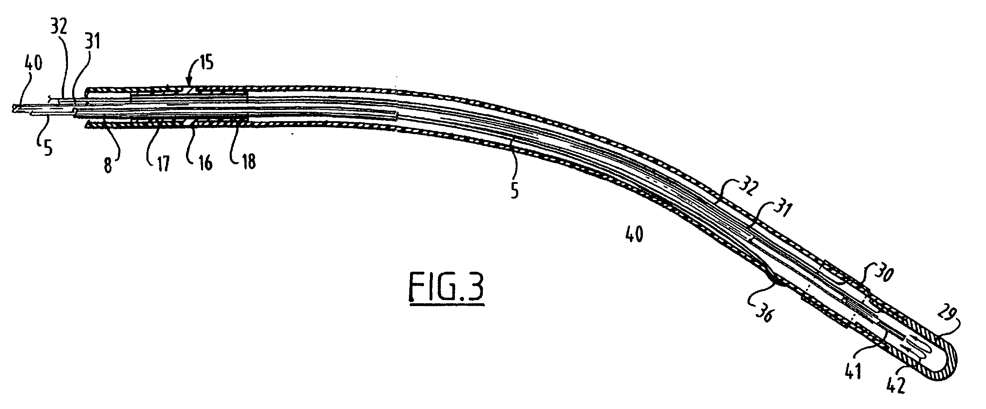

The pulling force exerted in this way is transferred to the end portion of the catheter body lying in between the connecting point 26 of the distal end of the pull element 5 with the catheter body and the stop 15. This portion of the catheter body therefore is compressed such that the end portion of the catheter takes the curved form als illustrated in Figure 3 in which also the same reference numerals indicate parts corresponding to those shown in Figures 1 and 2.

The embodiment of the catheter according to the invention shown in Figure 3 makes cryo-ablation possible of specific tissue areas which have been located by means of the electrical activity measurements as discussed above. The bending mechanism according to the invention however, can be embodied in the same way.

It will be clear that when pushing the engaging ring 24 further forward relative to the housing 22 the curve of the end portion is increased and vice versa.

Accordingly the person performing the treatment can accurately control the curve of the end portion of the catheter and manoeuvre the two electrodes 29, 30 in proper contact with the tissue to be measured.

The cryo-ablation embodiment of the catheter according to the invention as shown in Figure 3 is provided with an additional tubing 40 extending from the proximal end of the catheter through the lumen 4. In the distal end portion as shown in Figure 3 the tubing 40 is provided with a nozzle 41 having a passage with a restricted section relative to the section of the tubing 40 itself.

In a way known as such a high-pressure gas or liquid is supplied through the tubing 40 and exits the nozzle 41 in the catheter tip. There the fluid 42 expands which causes a significant decrease in temperature. In this way the tip portion 29 is cooled down, to a temperature which makes possible the cryo-ablation of tissue in contact therewith.

In this embodiment the tip portion 29 is made of a metallic material, which is thermally conductive as well as electrically conductive for making possible the electrical activity measurements indicated above.

Due to the bending mechanism according to the invention the tip portion 29 can be accurately positioned against tissues to be measures and ablated.

As the portion of the catheter in between the most distal and proximal positions of the catheter body where the sleeve 8 is connected to the basic body, for example by means of stops 10 and 15 remains free from compression forces, the catheter can be embodied with a thin wall, providing a relatively large cross-sectional area of the lumen 4. As furthermore the pull element 5 surrounded by the sleeve 8 takes up only a limited portion of the cross-sectional area of the lumen 4, the free cross-sectional area of the lumen 4 is large, providing a low flow resistance for the gases expanded in the tip portion 29. Accordingly, the pressure in the tip portion can remain low and fluid exiting from the nozzle 41 experiences a substantial pressure drop with a resulting large temperature drop.

Although not shown, a thermo-couple can be mounted inside the tip portion 29, connected to signal lines running to the proximal end. By means of this thermo-couple the temperature of the tip portion 29 can be controlled in a manner known as such.

In a further embodiment, not shown, the catheter body is provided with a brading the wires of which can act as signal lines in stead of the signal lines 31, 32 and signal lines from a thermal couple. This further increases the free sectional area of the lumen, making an improved cooling effect in the tip portion or a smaller diameter of the catheter with same cooling effect possible.

Although in the illustrated embodiments the sleeve 8 is connected to the basic body in only two places, it is obviously possible to do so in more places.

## Claims

1. Catheter (1) comprising a tube-like basic body (2) with a distal and a proximal end, at least one lumen (4), a flexible catheter-end-section (3) at the distal end and a pull element (5) extending through the lumen and connected with the basic body (2) close to the distal end which is surrounded by a sleeve (8) along at least part of its length, which is connected with the basic body (2) at least close to the proximal end of the basic body (2) and at the relatively proximal end of the end-section (3), **characterized in that**, the sleeve (8) is connected to an end-stop plug (10) at the proximal end of the basic body (2) and to a transition plug (15) at the relatively proximal end of the flexible end-section (3), said plugs comprising a central section (16) having substantially the same diameter as the basic body (2) and having, on at least one side, a cylindrical push-on section (17, 18) which has a smaller diameter and on to which an adjoining section of the tube-like basic body (2) has been pushed, said transition plug (15) having a push-on section on either side, one of which carries the flexible end-section (3).

2. Catheter as claimed in claim 1, wherein the sleeve (8) has a greater compression resistance in the longitudinal direction than the basic body (2).

3. Catheter as claimed in claim 1 or 2, wherein the length of the sleeve (8) in between the connecting points is a little greater than the length of the basic body (2) in between those connecting points.

4. Catheter as claimed in one of the previous claims, wherein the sleeve (8) surrounds the pull element (5) in a fitting manner.

5. Catheter as claimed in one of the previous claims which is of the thin-walled type with a large lumen (4), wherein the wall thickness of the basic body (2) is substantially a tenth of the diameter of the lumen.

6. Catheter as claimed in claim 5, being a cryo-ablation catheter.

## Patentansprüche

1. Katheter (1) mit einem schlauchartigen Grundkörper (2), der ein distales Ende und ein proximales Ende aufweist, mit wenigstens einem in Längsrichtung verlaufenden Kanal (4), mit einem flexiblen Katheter-Endabschnitt (3) am distalen Ende und mit einem Zugelement (5), das sich durch den Kanal erstreckt und mit dem Grundkörper (2) in der Nähe des distalen Endes verbunden und über wenigstens einen Teil seiner Länge durch eine Hülse (8) umschlossen ist, wobei diese Hülse mit dem Grundkörper (2) wenigstens in der Nähe des proximalen Endes des Grundkörpers (2) und in der Nähe des Endabschnitts (3) mit diesem verbunden ist,
**dadurch gekennzeichnet, dass** die Hülse (8) mit einem Endstopfen (10) am proximalen Ende des Grundkörpers (2) und mit einem Übergangsstopfen (15) an dem relativ proximalen Ende des flexiblen Endabschnitts (3) festgelegt ist, dass die Stopfen einen zentralen Abschnitt (16) mit im Wesentlichen dem gleichen Durchmesser wie der Grundkörper (2) aufweisen und auf wenigstens einer Seite einen zylindrischen Aufschiebeabschnitt (17, 18) besitzen, dessen Durchmesser kleiner ist und auf den ein benachbarter Abschnitt des schlauchartigen Körpers (2) aufgeschoben ist, wobei der Übergangsstopfen (15) auf beiden Seiten einen Aufschiebeabschnitt besitzt, von dem der eine den flexiblen Endabschnitt (3) trägt.

2. Katheter nach Anspruch 1, bei welchem die Hülse (8) einen größeren Kompressionswiderstand in Längsrichtung besitzt als der Grundkörper (2).

3. Katheter nach den Ansprüchen 1 oder 2, bei welchem die Länge der Hülse (8) zwischen den Verbindungspunkten etwas größer ist als die Länge des Grundkörpers (2) zwischen diesen Verbindungspunkten.

4. Katheter nach einem der vorhergehenden Ansprüche, bei welchem die Hülse (8) das Zugelement (5) mit engem Passsitz umschließt.

5. Katheter nach einem der vorhergehenden Ansprüche, der eine dünne Wandstärke mit einem großen Durchgangskanal (4) aufweist, wobei die Wanddicke des Grundkörpers etwa 1/10 des Durchmessers des Kanals beträgt.

6. Katheter nach Anspruch 5, welcher ein Kryoablations-Katheter ist.

## Revendications

1. Cathéter (1) comprenant un corps de base tubulaire ayant une extrémité distale et une extrémité proximale, au moins un lumen (4), une partie d'extrémité flexible de cathéter (3) à l'extrémité distale et un élément de tirage (5) traversant le lumen qui est relié au corps de base (2) à proximité de l'extrémité distale et qui est entouré par un manchon (8) le long d'au moins une partie de sa longueur, qui est relié au corps de base (2) au moins à proximité de l'extrémité proximale du corps de base (2) et à l'extrémité relativement proximale de la partie terminale (3), **caractérisé en ce que** le manchon (8) est relié à une fiche terminale d'arrêt (10) disposée à l'extrémité proximale du corps de base (2) et à une fiche de transition (15) disposée à l'extrémité relativement proximale de la partie d'extrémité flexible (3), lesdites fiches comprenant une partie centrale (16) ayant sensiblement le même diamètre que le corps de base (2) et possédant, sur au moins un côté, une partie cylindrique faisant poussoir, (17, 18) de petit diamètre et sur laquelle une partie contiguë du corps de base tubulaire (2) est poussée, ladite fiche de transition (15) possédant une partie faisant poussoir à chacun de ses côtés, dont une supporte la partie d'extrémité flexible (3).

2. Cathéter selon la revendication 1, dans laquelle le manchon (8) offre une résistance de compression supérieure dans la direction longitudinale à celle du corps de base (2).

3. Cathéter selon la revendication 1 ou 2, dans lequel la longueur du manchon (8) entre les points de connexion est légèrement supérieure à la longueur du corps de base (2) entre ces points de connexion.

4. Cathéter selon l'une quelconque des revendications précédentes, dans lequel le manchon (8) entoure l'élément de tirage (5) d'une manière qui permet l'emboîtement.

5. Cathéter selon l'une quelconque des revendications précédentes qui est du type à paroi mince avec un lumen (4) important, dans lequel l'épaisseur de la paroi du corps de base (2) est sensiblement égale au dixième du diamètre du lumen.

6. Cathéter selon la revendication 5, qui est un cathéter pour une cryo-ablation.
